# EUROPÄISCHE PATENTSCHRIFT

(11) **EP 0 381 786 B1**
(45) Veröffentlichungstag und Bekanntmachung des Hinweises auf die Patenterteilung: **20.04.1994**
(21) Anmeldenummer: 89102032.3
(22) Anmeldetag: 06.02.1989
(51) Int. Cl.: A61B 6/00, H04B 10/22

(54) **Optische Datenübertragungsvorrichtung**
Optical data transmission device
Dispositif optique pour la transmission de données

(43) Veröffentlichungstag der Anmeldung: 16.08.1990
(73) Patentinhaber: SIEMENS AKTIENGESELLSCHAFT, 80333 München (DE)
(72) Erfinder: Keller, Walter, Dipl.-Ing., D-8525 Marloffstein (DE)

(56) Entgegenhaltungen:
- DE-A- 3 530 939
- RESEARCH DISCLOSURE, Nr. 165, Januar 1978, Seite 5, Nr. 16503, Havant, GB; "Optoelectronic transmission system for rotary machines"

## Beschreibung

Die Erfindung betrifft eine optische Datenübertragungsvorrichtung gemäß dem ersten Teil des Patentanspruches 1.

Eine Datenübertragungsvorrichtung dieser Art kann bei einem Computertomographen zur Übertragung der vom Detektor erzeugten Daten verwendet werden. Der rotierende Teil ist dabei vom Drehkranz mit dem Röntgenstrahler und dem Detektor gebildet.

Bei einer Übertragungsvorrichtung der eingangs genannten Art in einem Computertomographen ist es bekannt, eine Reihe von Lichtsendern und eine Reihe von Lichtempfängern vorzusehen, wobei die Abstände der Lichtsender und der Lichtempfänger so gewählt sind, daß eine kontinuierliche Datenübertragung zwischen dem rotierenden Teil und dem feststehenden Teil erfolgt (DE-A-35 30 939).

Eine optische Datenübertragungsvorrichtung gemäß dem ersten Teil des Patentanspruches 1 ist durch das Dokument "Research Disclosure, Nr. 165, Jan. 1978, Seite 5, Nr. 16503, Havant, GB; "Optoelectronic transmission system for rotary machines" beschrieben. Bei dieser bekannten Datenübertragungsvorrichtung sind die Lichtsender und die Lichtempfänger auf ihren jeweiligen Trägern ortsfest angebracht.

Der Erfindung liegt die Aufgabe zugrunde, eine optische Datenübertragungsvorrichtung gemäß dem ersten Teil des Patentanspruches 1 so auszubilden, daß mit einer geringen Anzahl von Lichtsendern eine störungsfreie Datenübertragung sichergestellt ist.

Diese Aufgabe ist erfindungsgemäß gelöst durch den zweiten Teil des Patentanspruches 1. Bei der erfindungsgemäßen Datenübertragungsvorrichtung genügen bereits zwei Lichtsender in Verbindung mit einem einzigen Lichtempfänger zur störungsfreien Datenübertragung. Bei der erfindungsgemäßen Datenübertragungsvorrichtung ist sichergestellt, daß der Lichtempfänger in jeder Stellung der Datenübertragungsvorrichtung mindestens einen Lichtsender sieht und bei konstantem gesendetem Signal bei der Rotation ein konstantes Ausgangssignal liefert.

Die Erfindung ist nachfolgend anhand der Zeichnung näher erläutert. Es zeigen:
- Fig. 1: einen Computertomographen zur Erläuterung der Anwendung der Erfindung, und
- Fig. 2 und 3: zwei Ausführungsformen einer bei dem Computertomographen gemäß Fig. 1 anwendbaren optischen Datenübertragungsvorrichtung.

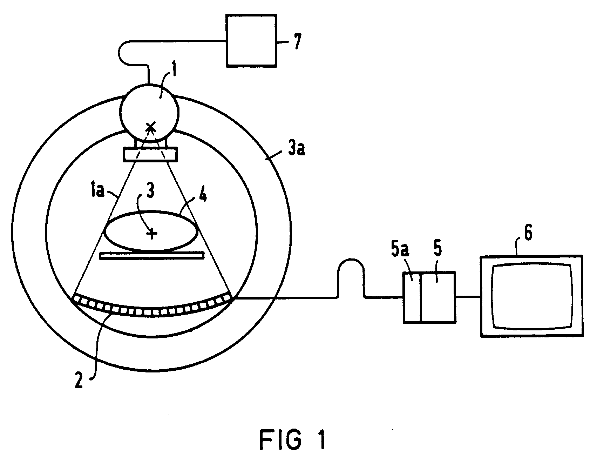

In der Fig. 1 ist ein Computertomograph mit einer Röntgenröhre 1 als Strahlenquelle und einem Strahlendetektor 2, der größenordnungsmäßig über 100, z.B. 512 einzelne Detektoren in einer Reihe angeordnet aufweist, dargestellt. Die Röntgenröhre 1 sendet ein fächerförmiges Strahlenbündel 1a aus, dessen Querschnittsausdehnung senkrecht zur untersuchten Schichtebene gleich der Schichtstärke und in der Schichtebene so groß ist, daß das ganze Aufnahmeobjekt von Strahlung durchsetzt wird. Der Strahlendetektor 2 ist um den Fokus der Röntgenröhre 1 gekrümmt. Die Meßanordnung 1, 2 ist um eine Achse 3, die etwa mit der Längsachse des Aufnahmeobjektes 4 zusammenfällt, mit Hilfe eines Drehrahmens 3a drehbar. Die Anzahl der Detektoren des Strahlendetektors 2 ist der gewünschten Bildauflösung entsprechend gewählt, so daß aufgrund einer Drehung der Meßanordnung 1, 2 von einem Computer 5 die Schwächungswerte einer Bildpunktmatrix der durchstrahlten Transversalschicht des Aufnahmeobjektes 4 berechnet und als Bild auf einem Sichtgerät 6 wiedergegeben werden können. Die Röntgenröhre 1 wird von einem Röntgengenerator 7 gespeist.

Jedem Detektorelement des Strahlendetektors 2 ist ein Meßkanal zugeordnet, der zum Computer 5 führt. In diesem Meßkanal sind Verstärkerschaltungen, Multiplexer und Analog-Digital-Wandler vorgesehen, die ein Datenerfassungssystem 5a bilden.

Eine besonders schnelle Anfertigung einer Vielzahl von Schnittbildern des Aufnahmeobjektes 4 ist möglich, wenn die Energie vom Röntgengenerator 7 zur Röntgenröhre 1 über Schleifringe und die Daten der Detektorelemente des Strahlendetektors 2 zum Datenerfassungssystem 5a ebenfalls über rotierende Datenübertragungsvorrichtungen übertragen werden. Dann ist nämlich eine Dauerrotation des Drehrahmens 3a und damit eine schnelle aufeinander folgende Abtastung einer Vielzahl von Schichten des Aufnahmeobjektes 4 möglich.

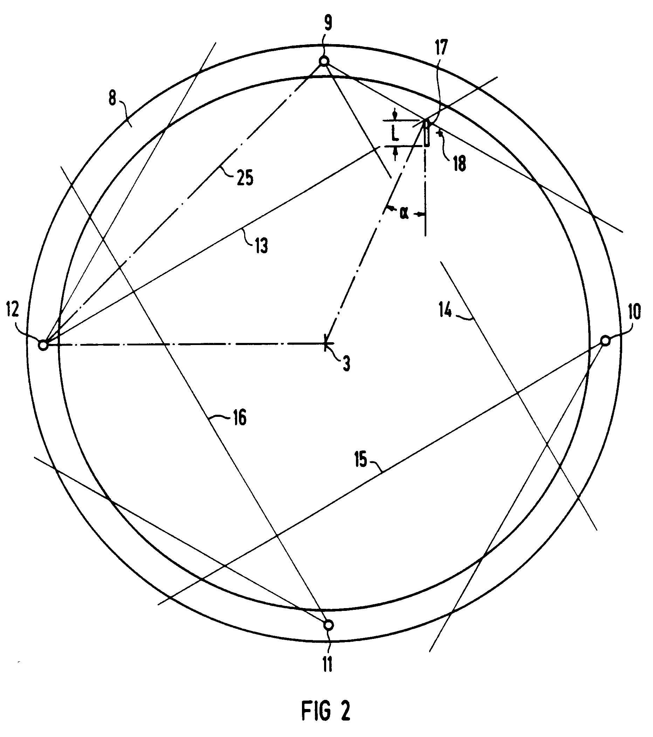

In der Fig. 2 ist eine optische Datenübertragungsvorrichtung zur kontaktlosen, optischen Übertragung der Daten der Detektorelemente des Strahlendetektors 2 dargestellt. Mit dem Drehrahmen 3a rotiert ein Rahmen 8, welcher symmetrisch auf seinem Umfang verteilt vier Lichtsender 9 bis 12 trägt, die dünne, fächerförmige Lichtbündel 13 bis 16 aussenden, welche der jeweils zu übertragenden Information entsprechend moduliert werden. Die Lichtbündel 13 bis 16 treffen auf einem Lichtempfänger 17 auf, der ein lichtelektrischer Wandler ist und die empfangenen Daten dem Datenerfassungssystem 5a zuführt. Er besitzt eine rechteckförmige lichtempfindliche Fläche, deren Längsausdehnung mit L bezeichnet ist. Der Winkel Alpha der Längsausdehnung L mit dem Radius wird so gewählt, daß der Lichtempfänger 17 in jeder Stellung des Rahmens 8 und damit der Lichtsender 9 bis 12 mindestens einen dieser Lichtsender 9 bis 12 sieht und daß bei konstantem gesendetem Signal bei der Rotation trotz der sich ändernden Abstände zwischen den Lichtsendern 9 bis 12 und dem Lichtempfänger 17 und der relativen Ausrichtung dieser Komponenten zueinander ein konstantes Ausgangssignal und damit eine störungsfreie Informationsübertragung erfolgt. Hierzu ist der Lichtempfänger 17 um eine parallel zur Achse 3 liegende Achse 18 schwenkbar und damit einstellbar.

Die Fig. 2 zeigt, daß bereits mit vier Lichtsendern 9 bis 12 und einem einzigen Lichtempfänger 17 eine störungsfreie Datenübertragung sichergestellt ist.

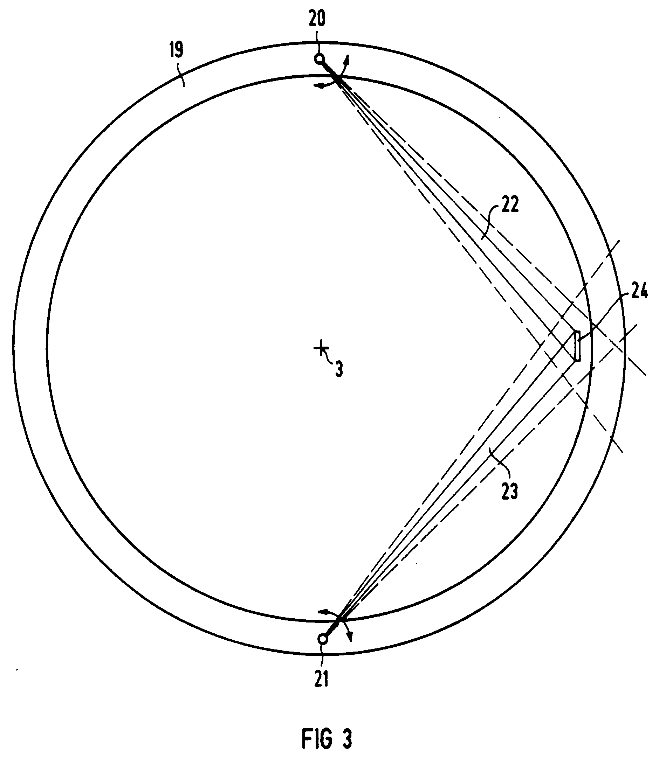

Das Ausführungsbeispiel gemäß Fig. 3 weist auf einem rotierenden Rahmen 19 nur zwei Lichtsender 20, 21 auf, die wieder zwei dünne, fächerförmige Lichtbündel 22, 23 aussenden, deren Fächerebenen wie bei dem Beispiel gemäß Fig. 2 in der durch den Rahmen 8 bzw. 19 definierten Ebene oder parallel dazu liegen. Ein Lichtempfänger 24 mit einer rechteckförmigen Empfangsfläche ist so angeordnet, daß diese Empfangsfläche senkrecht zum Radius liegt. Die Lichtsender 20, 21 sind in Richtung der gezeichneten Pfeile auf dem Rahmen 19 schwenkbar und werden bei der Drehung des Rahmens 19 um die Achse 3 automatisch so verschwenkt, daß ihre Lichtbündel auf den Lichtempfänger 24 nachgeführt werden. Dadurch ist es möglich, mit bereits zwei Lichtsendern zu erreichen, daß bei einer vollen Drehung des Rahmens 19 der Lichtempfänger 24 immer mindestens einen der Lichtsender 20, 21 sieht. Wird beispielsweise der Rahmen 19 im Uhrzeigersinn gedreht, so kann dabei der Lichtsender 21 um seine zur Achse 3 parallele Achse ebenfalls im Uhrzeigersinn verdreht werden, so daß das Lichtbündel 23 auf den Lichtempfänger 24 gerichtet bleibt.

Sowohl bei dem Ausführungsbeispiel gemäß Fig. 2 als auch gemäß Fig. 3 bilden die Zentralstrahlen der Lichtbündel 13 bis 16 bzw. 22, 23 mit dem entsprechenden Radius einen spitzen Winkel, so daß der Lichtempfänger 17 bzw. 24 immer mindestens einen Lichtsender 9 bis 12, 20, 21 sieht. Dieser Winkel ist für den Zentralstrahl 25 des Lichtbündels 13 in Fig. 2 eingezeichnet.

## Patentansprüche

1. Optische Datenübertragungsvorrichtung zur Datenübertragung zwischen einem rotierenden Teil (8, 19) und einem feststehenden Teil, bei der auf einem Teil (8, 19) eine Anzahl von Lichtsendern (9 bis 12, 20, 21) auf einem Kreis angeordnet ist, deren den zu übertragenden Daten entsprechend modulierte Strahlung von einem Lichtempfänger (17, 24) auf dem anderen Teil empfangen wird, wobei die Zentralstrahlen (25) mit dem Radius des die Lichtsender (9 bis 12, 20, 21) tragenden Teils (8, 19), welcher zum jeweiligen Lichtsender (9 bis 12, 20, 21) verläuft, jeweils einen Winkel bilden, **dadurch gekennzeichnet,** daß die Lichtsender (9 bis 12, 20, 21) fächerförmige Lichtbündel (13 bis 16, 22, 23) aussenden, deren Fächerebene in der durch den Kreis definierten Ebene oder parallel dazu liegt, daß der Lichtempfänger (17, 24) innerhalb des rotierenden Teiles (9 bis 12, 20, 21) angeordnet ist, daß die Lichtbündel (13 bis 16, 22, 23) nach innen gerichtet sind, und daß die Lichtsender (20, 21) auf dem rotierenden Teil (8, 19) während der Rotation um eine zur Drehachse (3) parallele Achse derart drehbar sind, daß ihre Lichtbündel (22, 23) auf den Lichtempfänger (24) nachgeführt werden.

2. Optische Datenübertragungsvorrichtung nach Anspruch 1, **dadurch gekennzeichnet,** daß der Lichtempfänger (17) um eine zur Drehachse (3) parallele Achse (18) einstellbar ist.

## Claims

1. An optical data-transmission arrangement for transmitting data between a rotating portion (8, 19) and a fixed portion, in which a number of light transmitters (9 to 12, 20, 21) are arranged on a circle on one portion (8, 19), the radiation of which transmitters, modulated in accordance with the data to be transmitted, is received by a light receiver (17, 24) on the other portion, with the central rays (25) each forming an angle with the radius of the portion (8, 19) carrying the light transmitters (9 to 12, 20, 21), which radius extends to the respective light transmitter (9 to 12, 20, 21), characterised in that the light transmitters (9 to 12, 20, 21) emit fan-shaped light beams (13 to 16, 22, 23), the fan plane of which lies in the plane defined by the circle or parallel thereto, in that the light receiver (17, 24) is arranged within the rotating portion (9 to 12, 20, 21), in that the light beams (13 to 16, 22, 23) are directed inwards and in that the light transmitters (20, 21) on the rotating portion (8, 19) are rotatable during the rotation about an axis parallel to the axis of rotation (3) in such a way that their light beams (22, 23) are directed onto the light receiver (24).

2. An optical data-transmission arrangement according to claim 1, characterised in that the light receiver (17) is adjustable about an axis (18) which is parallel to the axis of rotation (3).

## Revendications

1. Dispositif optique de transmission de données entre une pièce rotative (8, 19) et une pièce fixe, dans lequel, sur une pièce (8, 19), sont disposés sur un cercle un nombre d'émetteurs de lumière (9 à 12, 20, 21), dont le rayonnement modulé en fonction des données à transmettre est reçu, sur l'autre pièce par un récepteur de lumière (17, 24), les rayons centraux (25) formant un angle avec le rayon de la pièce (8, 19) qui porte les émetteurs de lumière (9 à 12, 20, 21), rayon qui s'étend jusqu'à chaque émetteur de lumière (9 à 12, 20, 21), caractérisé en ce que les émetteurs de lumière (9 à 12, 20, 21) émettent, sous forme d'éventail, des faisceaux lumineux (13 à 16, 22, 23), dont le plan est dans le plan défini par le cercle ou parallèle à ce plan, que le récepteur de lumière (17, 24) est disposé à l'intérieur de la pièce rotative (9 à 12, 20, 21), que les faisceaux lumineux (13 à 16, 22,23) sont dirigés vers l'intérieur, et que les émetteurs de lumière (20, 21), situés sur la pièce rotative (8, 9), sont susceptibles de tourner, pendant la rotation, autour d'un axe parallèle à l'axe de rotation (3), de telle sorte que leurs faisceaux lumineux (22, 23) sont orientés sur le récepteur de lumière (24).

2. Dispositif optique de transmission de données suivant la revendication 1, caractérisé en ce que le récepteur de lumière (17) est susceptible d'être réglé autour d'un axe (18) parallèle à l'axe de rotation (3).
